# EUROPEAN PATENT APPLICATION

(11) **EP 2 481 357 A2**
(43) Date of publication of application: **01.08.2012**
(21) Application number: 10819024.0
(22) Date of filing: 17.09.2010
(51) Int. Cl.: A61B 17/04, A61B 17/068

(54) **SUTURE APPARATUS HAVING SEWING FUNCTION**

(30) Priority: 22.09.2009 KR 20090089798
(71) Applicant: Rimscience Co., Ltd., Seoul 156-881 (KR)
(72) Inventor: YOON, Sang Jin, Seochu-gu, Seoul 137-925 (KR)
(74) Representative: Zardi, Marco
(86) International application number: PCT/KR2010/006448
(87) International publication number: WO 2011/037383

(57) **Abstract**

The present invention relates to a suture apparatus having a sewing function. More specifically, the present invention relates to a suture apparatus which allows an operator such as doctor or the like to suture human tissue or any other animal's body tissue accurately, quickly and easily. In accordance with one embodiment of the present invention, there is provided a suture apparatus which comprises a support, a surgical needle disposed with respect to the support, and a needle driving part for accommodating the surgical needle and operating the surgical needle, wherein the surgical needle operates with respect to the support, a suture thread from the surgical needle forms a plurality of rings according to the operation of the surgical needle, and the plurality of rings are sequentially connected.

## Description

### TECHNICAL FIELD

The present invention relates to a suture apparatus having a sewing function. More specifically, the present invention relates to a suture apparatus which allows an operator such as doctor or the like to suture human tissue or any other animal's body tissue accurately, quickly and easily.

### BACKGROUND ART

In a surgical operation, it is essential to suture various tissues, such as a serosa, a muscle, a fascia, a skin, a blood vessel, or the like of a human body. The main concern in the suturing process is shortening a time required for suturing so that fatigue of a medical practitioner is reduced, anesthesia time of a patient (or an animal subject to a surgical operation) is minimized, healing speed of patient's wounds is accelerated, and a loss of the patient's blood is minimized. In addition, in terms of beauty, minimizing scars resulting from the surgical operation may also be of important concern.

Conventionally, suturing is largely performed in a manner that a medical practitioner sews a tissue, which is a suturing target, through a manual operation by using an operation needle and a suture thread.

However, in such a conventional method, a time required for suturing is determined by the skill of the medical practitioner, thus an operation time may be lengthened according to circumstances. This may result in increasing the medical practitioner's fatigue, lengthening the anesthesia time of a patient, and causing a problem with a blood management, and the like. Also, the degree of scars according to suturing may be dependent upon how the stitches are uniform and dense, but suturing by the manual operation does not guarantee uniformity and enough density of the stitches.

### DISCLOSURE

### TECHNICAL PROBLEM

In order to solve the foregoing problems of the prior art, the applicant of the present invention invented a novel surgical suture apparatus, and filed a Korean Patent Application No. 2009-37128, which was already granted (the disclosure of which should be considered to be incorporated herein by reference in its entirety). The applicant of the present invention proposes a novel suture apparatus, which is based on a technical concept of the above surgical suture apparatus, and has a simpler structure as follows.

### TECHNICAL SOLUTION

It is, therefore, an object of the present invention to solve all of the aforementioned problems of the prior art.

It is another object of the present invention to provide a suture apparatus, wherein the suturing can be sophisticatedly and easily performing suturing.

It is still another object of the present invention to provide a suture apparatus capable of shortening a suture time.

It is still another object of the present invention to provide a suture apparatus capable of minimizing scars due to a suturing.

It is still another object of the present invention to provide a suture apparatus coupled to an external device such as a surgical robot system having robot arms or the like to perform automated suturing.

### ADVANTAGEOUS EFFECTS

In accordance with the present invention, a suture apparatus capable of sophisticatedly and easily performing suturing is provided.

In accordance with the present invention, a suture apparatus capable of shortening a suture time is provided.

In accordance with the present invention, a suture apparatus capable of minimizing scars due to a suturing is provided.

In accordance with the present invention, a suture apparatus coupled to an external device such as a surgical robot system having robot arms or the like to perform automated suturing is provided.

### DESCRIPTION OF DRAWINGS

The above objects and features of the present invention will become apparent from the following description of the preferred embodiments given in connection with the accompanying drawings, in which:
Fig. 1 is a view illustrating an overall configuration of a suture apparatus in accordance with an embodiment of the present invention.
Fig. 2 is a view illustrating the configuration of a suturing part in accordance with an embodiment of the present invention.
Fig. 3 is a view illustrating a suturing structure of a suture thread by the suture apparatus in accordance with an embodiment of the present invention.
Fig. 4 is a view illustrating a suturing process by the suture apparatus in accordance with an embodiment of the present invention.
Fig. 5 is a view illustrating the configuration of a suturing part in accordance with another embodiment of the present invention.
Fig. 6 is a view illustrating a suturing part employing a rotary unit.
Fig. 7 is a view illustrating the configuration of a suture apparatus in accordance with another embodiment of the present invention.
Fig. 8 is a view illustrating a suturing structure of a suture thread by the suture apparatus in accordance with another embodiment of the present invention.
Fig. 9 is a conceptual view of suturing in accordance with the embodiment of Fig 8.
Fig. 10 is a schematic view illustrating a suturing part in accordance with the embodiment of Fig 8.

### BEST MODE

Representative configurations for achieving the aforementioned objects of the present invention are presented as follows.

In accordance with one embodiment of the present invention, there is provided a suture apparatus comprising: a support; a surgical needle disposed with respect to the support; and a needle driving unit for accommodating the surgical needle and operating the surgical needle, wherein the surgical needle operates with respect to the support, a suture thread from the surgical needle forms a plurality of rings according to the operation of the surgical needle, and the plurality of rings are sequentially connected.

In addition, other configurations for implementing the present invention may be further provided.

### MODE FOR INVENTION

In the following detailed description, reference is made to the accompanying drawings that show, by way of illustration, specific embodiments according to which the present invention may be practiced. These embodiments are described in sufficient detail to enable those skilled in the art to practice the present invention. It is to be understood that the various embodiments of the present invention, although different, are not necessarily mutually exclusive. For example, a particular feature, structure or characteristic described herein in connection with an embodiment may be implemented within other embodiments without departing from the spirit and scope of the present invention. In addition, it is to be understood that the location or arrangement of individual elements within each disclosed embodiment may be modified without departing from the spirit and scope of the present invention. The following detailed description is, therefore, not to be taken in a limiting sense, and the scope of the present invention is defined only by the appended claims, appropriately interpreted, along with the full range of equivalents to which the claims are entitled. In the drawings, similar numerals refer to the same or similar elements throughout the several views.

Hereinafter, various preferred embodiments of the present invention will be described in detail with reference to the accompanying drawings so that a person skilled in the art can easily practice the present invention.

### [Embodiments]

The overall configuration and detailed components of the suture apparatus in accordance with the present invention may be similar in many parts to those of the surgical suture apparatus disclosed in Korean Patent Application No. 2009-37128 as mentioned above. Thus, hereinafter, the description will be made focusing on the characteristic features of the suture apparatus and the features required to be described again or additionally in accordance with the present invention.

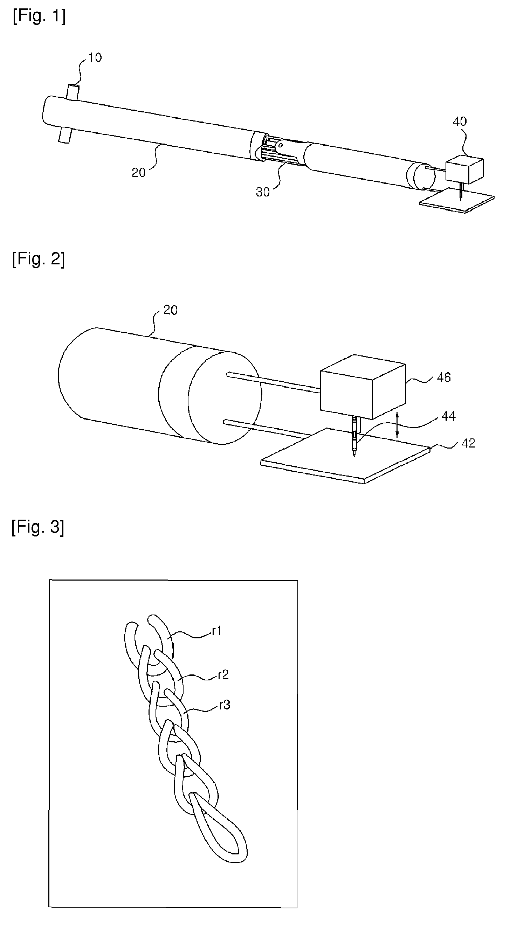

Fig. 1 is a view illustrating an overall configuration of a suture apparatus in accordance with an embodiment of the present invention.

Referring to Fig. 1, it can be seen that a suture apparatus may be configured to include a coupling part 10, a shaft part 20, an operating part 30, and a suturing part 40.

In accordance with an embodiment of the present invention, when the suture apparatus is coupled to an external device (not shown) such as a surgical robot system having a robot arm or any other surgical systems, the coupling part 10 may perform a function of receiving a certain control signal or certain control manipulation (e.g., a mechanical control manipulation) therefrom.

In accordance with an embodiment of the present invention, the shaft part 20 is an element for connecting the coupling part 10 and the suturing part 40. Here, one or more shafts may be provided with a joint element such as the operating part 30 to allow the suture apparatus to make a joint movement. In the interior of the shafts a wire or other control attachments for controlling the suturing part 40 as explained later may be included. Further, the shaft part 20 may serve to physically support the suture apparatus.

In accordance with an embodiment of the present invention, the operating part 30 may be disposed at an end or between the ends of one or more shafts of the shaft part 20 to perform a function of allowing the suture apparatus to make a joint movement and a function of determining the position and direction of the suturing part 40 in response to a certain control signal from an external device or an external control manipulation. The operating part 30 may enable the suturing part 40 and/or the shafts of the shaft part 20 to operate in a pitch direction and/or a yaw direction as necessary. In order to configure the operating part 30, principles of a joint part of various known surgical instruments may be employed.

Hereinafter, the suturing part 40 in accordance with various embodiments of the present invention will be described in detail.

Fig. 2 is a view illustrating a configuration of a suturing part (40) in accordance with an embodiment of the present invention.

Referring to Fig. 2, the suturing part 40, which is an element serving to suture a tissue, may include a support (or a lower support) 42, a surgical needle 44, and a needle driving unit 46 for accommodating the surgical needle 44 and operating the surgical needle 44 in a direction substantially perpendicular to the support 42.

Meanwhile, in the above or below detailed description, the support 42 of the suturing part 40 in accordance with the present invention is illustrated to be mainly the lower support 42, but it should be understood that the support 42 facing the surgical needle 44 may be disposed above the surgical needle 44 or in any other side of the surgical needle 44. Further, in the above or below detailed description, it is illustrated and described that the surgical needle 44 in accordance with the present invention operates in the direction substantially perpendicular to the support 42, but it should be appreciated that the scope of the present invention is not limited to the angle of the surgical needle with respect to the support 42 so long as suturing is performed according to the technical concept of the present invention.

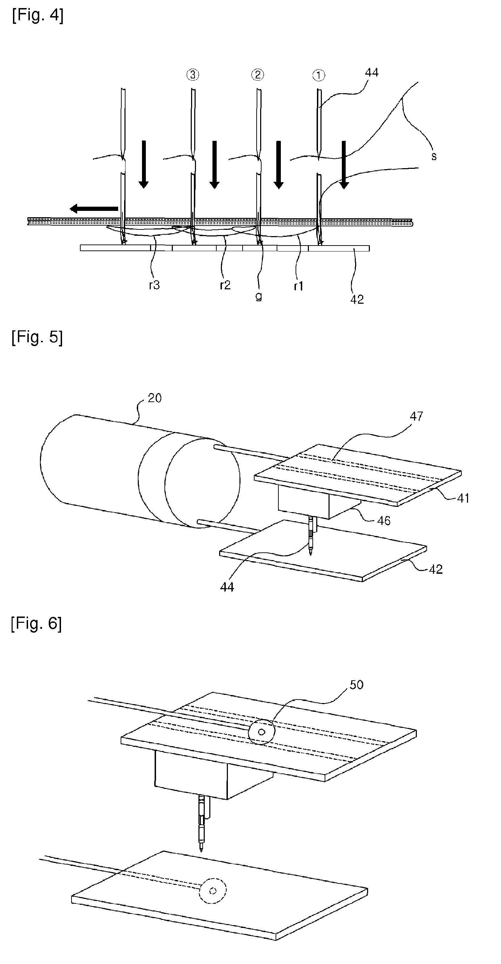

Fig. 3 is a view illustrating a suturing structure of a suture thread by the suture apparatus in accordance with an embodiment of the present invention, and Fig. 4 is a view illustrating a suturing process by the suture apparatus in accordance with an embodiment of the present invention.

Referring to Figs. 2 to 4, the suture apparatus in accordance with the present invention has structural characteristics in that it basically does not require a suture thread supplied from the support 42. That is, in the suturing unit 40 in accordance with an embodiment of the present invention, while the surgical needle 44 is operating up and down according to driving of the needle driving unit 46, a suture thread caught in the surgical needle 44 is entangled by itself, thus capable of performing suturing. More specifically, while the surgical needle 44 performs a vertical operation (movement) of one period, the surgical needle 44 penetrates a tissue to be sutured to form rings r1, r2, and r3 of the suture thread s at the opposite side thereof, and in this case, as the rings r2 and r3 are inserted into the first formed rings r1 and r2, respectively, the rings r1, r2, and r3 can be sequentially connected.

This will be further descried with reference to Figs. 3 and 4. The surgical needle 44, while making a first vertical operation ①, may penetrate a tissue to form a first ring r1. Thereafter, the needle driving unit 46 may move the surgical needle 44 by a certain distance in a direction in which suturing is made (that is, in a direction of the horizontal arrow illustrated in Fig. 4). And then, the surgical needle 44, while making a second vertical operation ②, may form a second ring r2 and simultaneously insert the second ring r2 into the first ring r1. Thereafter, the needle driving unit 46 may move the surgical needle 44 again by a certain distance in a direction in which suturing is made. Subsequently, the surgical needle 44, while making a third vertical operation ③, may form a third ring r3 and insert the second ring r3 into the first ring r2. In this manner, the suture thread s continuously forms the rings r1, r2, and r3 and the rings r1, r2, and r3 are sequentially connected, thus performing suturing.

Meanwhile, in such a case, a locking part g may be formed on the support 42 where the suture thread s is caught as it approaches the support 42 along with the surgical needle 44. While the surgical needle 44 approaches the support 42 and then moves away from the support 42, the locking part g may catch and fix a portion of the suture thread s, thus allowing the rings r1, r2, and r3 to be formed according to the operation of the surgical needle 44. Meanwhile, a plurality of locking parts g may be formed on the support 42 as necessary, and the intervals between the locking parts g may be freely determined as necessary. Further, such a locking part g may be transferred in a direction in which suturing is made (e.g., in the direction of the horizontal arrow illustrated in Fig. 4) along with the support 42 to allow the rings r1, r2, and r3 to be smoothly formed.

By repeating the foregoing process, suturing can be made only with the suture thread s supplied from the surgical needle 44 without a suture thread supplied from the support 42. The sutured state according to the suturing may be basically maintained by frictional force between the suture thread s and the tissue, but here, a knot forming means may be additionally used to allow the finally formed ring to serve as a so-called knot.

In accordance with the present invention, since a configuration for supplying a suture thread from the support 42 is not required, the overall configuration of the suture apparatus can be simplified. Thus, the suture apparatus can be easily fabricated, reducing fabrication costs.

In accordance with an embodiment of the present invention, in the suturing part 40, the needle driving unit 46 may be operated by a control signal from an external device or a controlling manipulation. For example, as the medical practitioner manipulates the external device, the surgical needle 44 accommodated in the needle driving unit 46 may operate in a direction substantially perpendicular to the support 42. Further, for example, as the medical practitioner manipulates the external device, the needle driving unit 46 and/or the support 42 may be driven in a direction in which suturing is made (in this connection, if necessary, it may be configured such that when the length of the shaft part 20 extends or contracts, the needle driving unit 46 and/or the support 42 operates accordingly).

Meanwhile, for the suture apparatus in accordance with the present invention, it should be appreciated that a configuration in which a progress mode of suturing is an automatic mode or a semi-automatic mode, a configuration in which intervals of stitches and/or a suturing rate are determined or set, a configuration in which the overall structure or dimensions of the suture apparatus is altered as necessary to fit an invasive surgery, or the like may be employed with reference to descriptions of Korean Patent Application No. 2009-37128.

Meanwhile, a suture apparatus in accordance with another embodiment of the present invention may include the coupling part 10, the shaft part 20, and the operating part 30, as shown in Fig. 2, and here, the suture apparatus in accordance with another embodiment of the present invention may be configured to include a slightly modified suturing part 40. Fig. 5 is a view illustrating the configuration of the suturing part 40 in accordance with another embodiment of the present invention.

Referring to Fig. 5, it can be seen that the suturing part 40 includes an upper support 41 and a lower support 42, which face each other, the surgical needle 44, and the needle driving unit 46 accommodating the surgical needle 44 and serving to operate the surgical needle 44 in a direction substantially perpendicular to the lower support 42. Further, the needle driving unit 46 may be disposed on the upper support 41 and configured to move along a rail 47 provided on the upper support 41 within a certain range.

Fig. 6 is a view illustrating the suturing part 40 employing a rotary unit 50. As shown in Fig. 6, the suturing part 40 may be coupled with a certain rotary unit 50. With reference to Fig. 6, the rotary unit 50 may enable the suturing part 40 to rotate within a certain angle range.

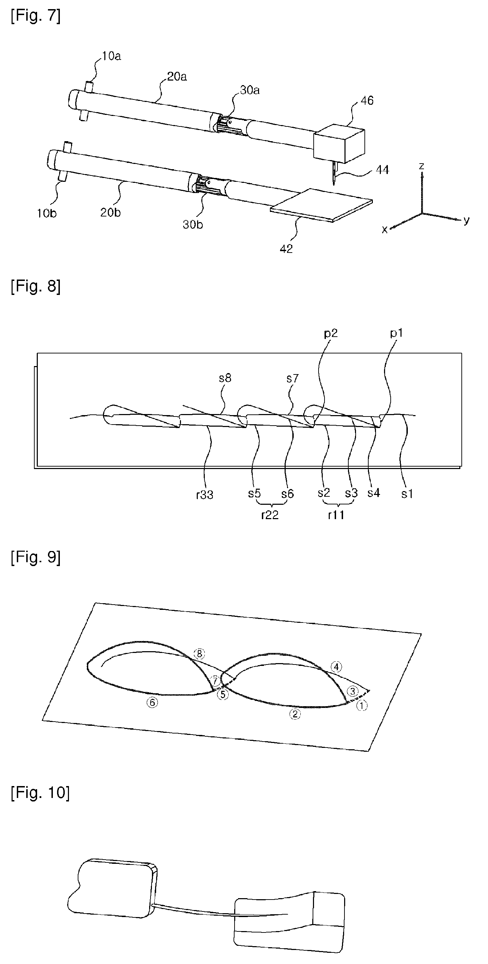

Fig. 7 is a view illustrating the configuration of a suture apparatus in accordance with another embodiment of the present invention. As shown in Fig. 7, the suture apparatus in accordance with the present invention to the present embodiment may have a so-called separation type configuration. That is, the suture apparatus may be configured to include a first coupling part 10a, a second coupling part 10b, a first shaft part 20a, a second shaft part 20b, a first operating part 30a, a second operating part 30b, the lower support 42, and the needle driving unit 46 for accommodating the surgical needle 44.

In accordance with the present embodiment, an upper portion of the suturing part 40 including the surgical needle 44 and the needle driving unit 46 may form a single separated module of the suture apparatus along with the first coupling part 10a, the first shaft part 20a, and the first operating part 30a as illustrated in Fig. 7, and the lower support 42 corresponding to a lower portion of suturing part 40 may form another separated module of the suture apparatus along with the second coupling part 10b, the second shaft part 20b, and the second operating part 30b as illustrated in Fig. 7. Here, the first coupling part 10a, the first shaft part 20a, and the first operating part 30a may be separated from the second coupling part 10b, the second shaft part 20b, and the second operating part 30b.

In the suture apparatus having the separate type configuration in accordance with the present embodiment, the respective modules may approach a tissue to be sutured in various directions. Further, the respective modules may freely perform a joint operation, may be controlled by different external devices, and may be advantageously used to suture tubular internal organs or to perform suturing by using a surgical port.

Regarding the modified configuration of the suture apparatus in accordance with the present invention described with reference to Figs. 5 to 7, Korean Patent Application No. 2009-37127 may be further referred to.

Meanwhile, Fig. 8 is a view illustrating a suturing structure of a suture thread by the suture apparatus in accordance with another embodiment of the present invention, and Fig. 9 is a conceptual view of suturing in accordance with the embodiment of Fig 8.

Referring to Figs. 8 and 9, the suture apparatus in accordance with the present embodiment may have a configuration basically similar to that of the suture apparatus illustrated in Fig. 2. That is, the suture apparatus in accordance with the present embodiment also have structural characteristics in that it does not require a suture thread supplied from a support (not shown) and a suture thread caught in a surgical needle (not shown) (preferably, caught in a tip portion) is entangled by itself, thus performing suturing. In this case, the surgical needle may have a bent shape similar to that for a conventional manual surgical operation. Further, the support may also have a bent shape for allowing the surgical needle to penetrate a tissue twice or the like, as explained hereinafter (in this connection, Fig. 10 may be further referred to).

First, in the course of penetrating a tissue to be sutured twice, that is, downwardly and then upwardly, the surgical need (or the tip portion thereof) may form a suture thread portion p1 (i.e., a portion ① in Fig. 9) distinguished from a suture thread portion s1 of an upper surface of the tissue by the suture thread caught in the surgical needle (in Figs. 8 and 9, the suture thread portion indicated by the solid line belongs to an upper surface of the tissue and the suture thread portion indicated by the dotted line belongs to a lower surface of the tissue). And then, a certain locker (not shown) operates to pull a small ring of the suture thread held by the surgical needle on the tissue after forming the suture thread portion p1 (here, the suture thread portion p1 may eventually include two lines because the surgical needle retreats as explained hereinafter), to form a ring r11 (i.e., a portion ② in Fig. 9) composed of the suture thread portions s2 and s3. Thereafter, the surgical needle retreats the tissue downwardly and then upwardly (during this process, a portion ③ in Fig. 9 is formed) to form a suture thread portion s4 (i.e., a portion ④ in Fig. 9) on the upper surface of the tissue. In forming the suture thread portion s4, the needle driving unit (not shown) may move the surgical needle by a certain distance in a direction in which the suturing is made (that is, from the right to the left in Fig. 8). In this case, the suture thread portion s4 may be formed at an upper location than a portion of the ring r11 in the perspective of the tissue.

And then, according to the operation of the surgical needle and the needle driving unit in the same manner as described above, a suture thread portion p2 (i.e., a portion ⑤ in Fig. 9) on a lower surface of the tissue may be formed, a ring r22 (i.e., a portion ⑥ in Fig. 9) comprised of suture thread portions s5 and s6 may be formed, and then, a suture thread portion s7 (i.e., a portion ⑧ in Fig. 9) may be formed. In this case, the suture thread portion s7 may be formed at an upper location than a portion of the ring r11 or a portion of the ring r22 in the perspective of the tissue to indirectly connect the ring r11 and the ring r22.

Thus, in the present embodiment, suturing can also be performed by sequentially connecting the rings of the suture thread from the surgical needle. In particular, such suturing may be advantageously used to suture a multilayer tissue.

While embodiments of the invention has been shown and described, it will be understood by those skilled in the art that various changes and modifications may be made without departing from the spirit and the scope of the invention as defined in the following claims.

## Claims

1. A suture apparatus comprising:
a support;
a surgical needle disposed with respect to the support; and
a needle driving unit for accommodating the surgical needle and operating the surgical needle,
wherein the surgical needle operates with respect to the support,
a suture thread from the surgical needle forms a plurality of rings according to the operation of the surgical needle, and
the plurality of rings are sequentially connected.

2. The suture apparatus as claimed in claim 1, wherein the support includes at least one locking part.

3. The suture apparatus as claimed in claim 1, wherein the surgical needle operates in a direction substantially perpendicular to the support.

4. The suture apparatus as claimed in claim 1, wherein the plurality of rings are connected such that one ring is inserted into a ring adjacent thereto.

5. The suture apparatus as claimed in claim 1, wherein the needle driving unit or the support move in a direction parallel to a tissue to be sutured.

6. The suture apparatus as claimed in claim 1, wherein the surgical needle has a bent shape.

7. The suture apparatus as claimed in claim 1, further comprising at least one locker.

8. The suture apparatus as claimed in claim 1, wherein the plurality of rings are connected such that one ring is indirectly connected to a ring adjacent thereto.
